# FASCICULE DE BREVET EUROPEEN

(11) **EP 2 340 059 B1**
(45) Date de publication et mention de la délivrance du brevet: **25.07.2012**
(21) Numéro de dépôt: 08864626.0
(22) Date de dépôt: 30.09.2008
(51) Int. Cl.: A61B 17/02, A61L 15/28, A61L 15/44, A61L 15/46, A61B 19/08, A61L 31/04, A61L 28/00, A61L 26/00

(54) **AGENT ANTIBACTÉRIEN ET DISPOSITIF ACTIF DES BERGES D'INCISION INTÉGRANT UN TEL AGENT ANTIBACTÉRIEN.**
ANTIBAKTERIELLES MITTEL UND VORRICHTUNG ZUM AKTIVEN SCHUTZ VON SCHNITTRÄNDERN MIT EINEM SOLCHEN ANTIBAKTERIELLEN MITTEL
ANTIBACTERIAL AGENT, AND DEVICE USED FOR ACTIVE PROTECTION OF INCISION MARGINS AND INCORPORATING SUCH AN ANTIBACTERIAL AGENT

(30) Priorité: 20.12.2007 FR 0708933; 18.01.2008 FR 0850316
(43) Date de publication de la demande: 06.07.2011
(73) Titulaire: Touati, Gilles, 80000 Amiens (FR)
(72) Inventeur: Touati, Gilles, 80000 Amiens (FR)
(74) Mandataire: Roman, Alexis
(86) Numéro de dépôt international: PCT/FR2008/051760
(87) Numéro de publication internationale: WO 2009/080922

(56) Documents cités:
- US-A1- 2004 103 903
- US-A1- 2005 249 791
- US-A1- 2006 065 275
- SPANGLER D, ROTHENBURGER S, NGUYEN K, JAMPANI H, WEISS S, BHENDE S: "In vitro antimicrobial activity of oxidized regenerated cellulose against antibiotic-resistant microorganisms" SURGICAL INFECTIONS, vol. 4, no. 3, 2003, pages 255-262, XP008093557

## Description

### Domaine technique de l'invention.

La présente invention a pour objet un agent antibactérien comportant comme produit actif un tissu hémostatique de contact ayant un pH acide combiné à de la polyvidone iodée. Elle concerne également un dispositif de protection actif des berges d'incision intégrant un tel un agent antibactérien.

L'invention concerne le domaine technique des agents antibactériens destinés à être utilisés sur l'homme et/ou sur les animaux dans le but de protéger et/ou de traiter une plaie et/ou une incision contre des infections bactériennes. Elle concerne également le domaine technique des accessoires de chirurgie permettant de protéger les berges d'incision lors d'une intervention chirurgicale. L'invention concerne plus particulièrement le domaine technique des champs de bordure chirurgicaux.

### État de la technique.

Les tissus hémostatiques de contact sont habituellement utilisés pour le traitement des hémorragies non contrôlables par les techniques traditionnelles, qu'elles soient cutanées ou internes (hémorragies capillaires et veineuses ou artérielles ; saignements en nappes ; chirurgie cardiaque et vasculaire ; neurochirurgie ; urologie ; ...).

De par leur conception, ces tissus hémostatiques ont généralement un pH acide de sorte qu'ils ont indirectement une activité antibactérienne. Toutefois, les fabricants préconisent de ne pas l'utiliser à des fins antibactériennes. En pratique, l'activité antibactérienne doit être mise en oeuvre par un agent spécialement prévu à cet effet.

La polyvidone iodée (ou polyvinylpyrrolidone iodée) est un agent externe qui libère de l'iode inorganique agissant directement sur les protéines cytoplasmiques à l'état d'iode libre. La polyvidone iodée est connue pour ses propriétés antibactériennes, antiseptiques, ou encore antifongiques. Bien qu'extrêmement efficace, la polyvidone iodée a une utilisation limitée, car elle ne permet pas de traiter toutes les souches de germes pouvant être à l'origine de résistances croisées antiseptiques/antibiotiques.

On connaît par le document SPANGLER et al: « In vitro antimicrobial activity of oxidized regenerated cellulose against antibiotic-resistant micro-organisms » SURGICAL INFECTIONS, vol.4, N°3, 2003, pages 255-262, le résultat d'une étude in-vitro portant sur l'activité antimicrobbienne de matériaux de cellulose oxydée régénérée. Cette étude démontre l'efficacité de la cellulose oxydée régénérée sur des micro-organismes résistants à plusieurs antibiotiques, et plus particulièrement l'importance d'un pH acide. Cette étude ne fait toutefois pas mention de la combinaison antimicrobienne entre la cellulose oxydée régénérée et de la polyvidone iodée.

Un premier problème technique que vise à résoudre l'invention est de proposer un nouvel agent ayant une activité antibactérienne supérieure à celle d'un tissu hémostatique et supérieure à celle de la polyvidone iodée, de manière à pouvoir traiter un large panel de souches de germes et en particulier certaines résistantes aux antibiotiques.

Également, lors des opérations de chirurgie, il est courant d'employer des champs de bordure chirurgicaux équipés de fenêtres d'incision au travers desquelles sont réalisés les actes chirurgicaux. Ces champs se placent sur la peau du patient et ont pour but d'isoler et de protéger la zone d'incision contre toute contamination. Ils forment une barrière efficace entre le corps du patient et l'atmosphère du bloc opératoire.

Les champs de bordure chirurgicaux habituellement employés sont fabriqués à partir d'un matériau stérile souple non tissé. Généralement, la face inférieure en contact avec la peau du patient est apte à absorber les sécrétions corporelles telles que les saignements tandis que la face supérieure est imperméable aux fluides. Il est courant d'employer des champs cutanés chirurgicaux iodés ou imprégnés d'une substance antimicrobienne, ayant un effet rémanent sur les microbes présents sur la peau du patient (essentiellement les staphylocoques cutanés). De tels champs sont par exemple décrits dans les documents WO 2005/110082 (3M), WO 00/71183 (3M), EP 0.812.893 (MEDICAL CONCEPTS DEVELOPMENT), EP 0.240.097 (SURGIKOS), EP 0.136.900 (SURGIKOS) ou encore FR 2.012.584 (T.J. SMITH et al). Ces champs peuvent être adhésifs de manière à parfaitement rester en place tout au long de l'intervention et border au plus près la zone d'incision.

Bien que couramment employés, ces champs de bordure chirurgicaux ne sont pas totalement efficaces dans la mesure où ils ne sont que cutanés et ne protègent pas les berges d'incision. En effet, dès que la peau est incisée, un saignement même minime de ces tissus et l'exposition prolongée à l'air font que ces berges d'incision présentent un risque de voir se développer des infections, des abcès ou des hématomes tout à fait néfastes pour la santé du patient.

Pour tenter de résoudre ce problème technique, on connaît par le document WO 99/03416 (MEDICAL CREATIVE TECHNOLOGIES), l'utilisation d'un élément tubulaire souple rétractable que l'on positionne autour des berges d'incision. Cet élément tubulaire est toutefois difficile à mettre en place par le praticien, car sa manipulation est complexe. En outre, il est nécessaire de prévoir des éléments tubulaires de différents diamètres de façon à pouvoir s'adapter à la taille de l'incision réalisée, ce qui est peu pratique et coûteux.

Le document US 4.089.331 (THE KANDALL COMPANY) tente également d'apporter une solution en proposant d'utiliser une compresse stérile rapportée à l'extrémité d'un drap chirurgical, le long du bord de la fenêtre d'incision. Cette compresse est aménagée de manière à être dans une position de rangement avant l'incision - rangée dans une poche spécifique ou rabattue sur le dessus du drap - et dans une position venant recouvrir les berges d'incision après l'incision. La conception de ce dispositif apparaît être particulièrement complexe du fait qu'il faille prévoir une poche de rangement ou un dispositif permettant de maintenir la compresse en position rabattue. De plus, la longueur de la compresse ne peut être facilement ajustée de sorte que ladite compresse peut gêner l'intervention du praticien lorsqu'elle n'est pas adaptée à la profondeur de l'incision. Également, cette compresse n'est pas particulièrement efficace contre les infections ou contre la formation d'abcès ou d'hématomes.

Le document GB 2.221.620 (Johnson & Johnson) divulgue un pansement comprenant un substrat fibreux dont la surface est recouverte d'un alginate acceptable en pharmacologie. Ce type de pansement n'est toutefois pas spécifiquement adapté pour protéger les berges d'incision et éviter le développement des infections lors d'une intervention chirurgicale.

Les champs de bordure chirurgicaux existants ne sont pas totalement efficaces dans la mesure où ils ne permettent pas d'assainir efficacement le site opératoire. En effet, dès que la peau est incisée, des secrétions d'incision apparaissent (saignements et/ou secrétions corporelles) qui sont susceptibles d'endommager les berges d'incision et causer des infections de paroi et/ou du site opératoire, notamment des infections nosocomiales. De plus, ces sécrétions masquent certaines parties du site opératoire et donc entravent le bon déroulement de l'intervention chirurgicale. Il est donc nécessaire d'évacuer les sécrétions d'incision, généralement en utilisant des compresses absorbantes que l'on change régulièrement une fois qu'elles sont totalement imbibées. L'absorption des sécrétions par compresses induit des manipulations supplémentaires autour du site opératoire susceptibles de gêner le praticien dans l'exécution des actes chirurgicaux.

La compresse stérile rapportée à l'extrémité du drap chirurgical décrit dans le document US 4.089.331 cité précédemment, ne permet pas d'absorber efficacement les sécrétions d'incision. En effet, dès que la compresse est totalement imbibée, l'absorption des sécrétions ne peut plus être réalisée de sorte qu'il faille continuer à utiliser des compresses absorbantes supplémentaires et donc continuer à effectuer des manipulations supplémentaires autour du site opératoire.

On connaît par le document WO2003/018098 (KCI LICENSING INC) un système destiné à accélérer la cicatrisation d'un tissu difficilement cicatrisable. Ce système comprend un tampon poreux introduit dans une plaie ainsi qu'un pansement étanche à l'air fixé sur ce tampon, d'où l'obtention d'une fermeture hermétique sur la plaie. Une extrémité proximale d'un conduit peut être raccordée au pansement, une extrémité distale de ce conduit pouvant être raccordée à une source de dépression, telle qu'une pompe électrique logée dans un boîtier portatif , ou à une prise murale de vide. Un collecteur installé sur le conduit permet de retenir les exsudats aspirés à partir de la plaie pendant l'application d'une dépression. Bien qu'efficace, ce système est complexe et recouvre entièrement la plaie de sorte que le praticien n'a aucun accès au site d'incision. De plus, le système décrit dans le document WO2003/018098 (KCI LICENSING INC) ne protège pas la plaie contre d'éventuelles infections nosocomiales.

Face aux inconvénients de l'art antérieur et en particulier ceux du dispositif décrit dans US 4.089.331 (THE KANDALL COMPANY), le second problème technique que vise à résoudre l'invention est de protéger efficacement, rapidement et durablement les berges d'incision sans que le praticien n'ait à effectuer des manipulations complexes.

Un troisième problème technique que vise à résoudre l'invention est de proposer un dispositif du type décrit dans US 4.089.331 (THE KANDALL COMPANY) capable de protéger les berges d'incision de manière régulière durant tout le temps de l'opération.

Un quatrième problème technique que vise à résoudre l'invention est de proposer un dispositif du type décrit dans US 4.089.331 (THE KANDALL COMPANY) capable d'assainir efficacement, régulièrement et sans gène pour le praticien, les berges d'incision ainsi que le site opératoire, durant tout le temps de l'intervention chirurgicale.

Encore un autre but de l'invention est de proposer un dispositif de protection des berges d'incision permettant de prévenir les infections de paroi et/ou du site opératoire, et notamment prévenir les infections nosocomiales.

### Divulgation de l'invention.

Concernant le premier problème technique, le demandeur a maintenant mis en évidence de manière surprenante que la combinaison de cellulose oxydée régénérée ou non régénérée avec de la polyvidone iodée donnait une inhibition bactérienne supérieure à la simple utilisation de la cellulose oxydée régénérée ou non régénérée isolée ou de la polyvidone iodée isolée. Il s'agit donc d'un effet synergique qui aboutit à la constitution d'un nouvel agent à la fois hémostatique et antibactérien très puissant.

La solution proposée par l'invention pour résoudre le second problème technique est un dispositif de protection actif des berges d'incision destiné à éviter le développement des infections, comportant un champ de bordure chirurgical dans lequel est aménagée une fenêtre d'incision totale ou partielle, et dans lequel :
- la face interne des bordures de la fenêtre d'incision est recouverte d'une première couche d'un matériau destiné à absorber les secrétions d'incision et d'une seconde couche imprégnée d'un produit hémostatique antiseptique ayant pour but d'éviter le développement des infections,
- la seconde couche vient couvrir partiellement la première couche, ladite première couche étant plus large que ladite seconde couche,
- la seconde couche est aménagée de manière à ce qu'elle puisse venir au contact des berges d'incision et recouvre la partie supérieure et la partie inférieure de l'incision lors de la mise en place d'un écarteur,
- la seconde couche est un tissu de cellulose oxydée régénérée ou non régénérée combinée à de la polyvidone iodée.

Ces caractéristiques techniques permettent au praticien de disposer directement les bordures de la fenêtre d'incision au niveau des berges d'incision pour protéger efficacement ces dernières.

Le troisième problème technique est résolu lorsque la seconde couche est associée à un élément permettant d'imprégner, à la demande, le tissu hémostatique de contact avec de la polyvidone iodée. En effet, le praticien a ainsi la possibilité de ré-imprégner le tissu hémostatique avec de la polyvidone iodée, lorsque la quantité de cette dernière diminue au cours de l'opération.

La solution proposée par l'invention pour résoudre le quatrième problème technique est un dispositif de protection des berges d'incision comportant un champ de bordure chirurgical dans lequel est aménagée une fenêtre d'incision totale ou partielle, les bordures de ladite fenêtre d'incision étant aménagées de manière à ce qu'elles puissent venir au contact des berges d'incision. Conformément à l'invention, la face interne des bordures de la fenêtre d'incision est recouverte d'une couche d'un matériau destiné à absorber les secrétions d'incision, ladite couche étant associée à une tubulure reliée à un organe permettant d'aspirer lesdites secrétions absorbées.

Ces caractéristiques techniques permettent non seulement d'assécher les berges d'incision, mais encore d'évacuer de façon simple et régulière les sécrétions d'incision du site opératoire. Le demandeur a également pu constater qu'un tel dispositif permettait de prévenir efficacement les infections de paroi et/ou du site opératoire, et notamment les infections nosocomiales.

### Présentation des dessins.

D'autres avantages et caractéristiques de l'invention apparaîtront mieux à la lecture de la description d'un mode de réalisation préférée qui va suivre, en référence aux dessins annexés, réalisés à titre d'exemples indicatifs et non limitatifs et sur lesquels :

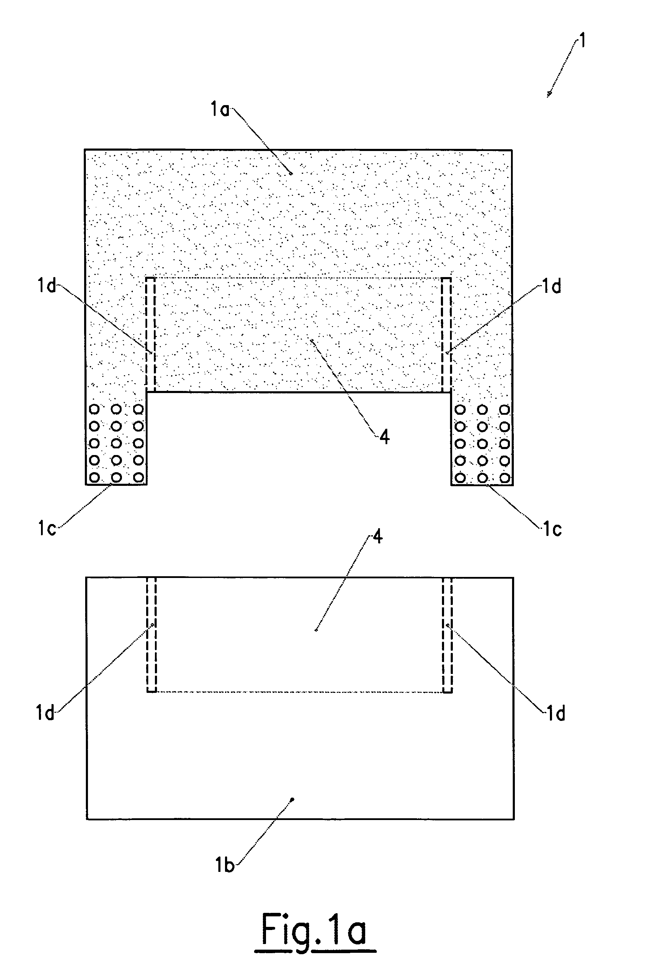

- la figure 1a est une vue schématique montrant les différents éléments constitutifs du champ de bordure chirurgicale conforme à l'invention,
- la figure 1 b est une vue schématique montrant les différents éléments constitutifs de la figure 1a aménagés de manière à former le champ de bordure chirurgicale conforme à l'invention,
- la figure 2 est une vue schématique montrant, de face, l'agencement des différents éléments constitutifs de la partie femelle du champ de bordure chirurgicale conforme à l'invention,
- la figure 3 est une vue en coupe selon A-A de la partie femelle du champ de bordure chirurgical représenté sur la figure 2,
- la figure 4 est une vue schématique montrant, en coupe, le dispositif de protection de l'invention mis en place contre les berges d'incision,
- la figure 5 est une vue schématique montrant, de face, la partie femelle du champ de bordure chirurgicale équipée des éléments d'aspiration des secrétions d'incision,
- la figure 6 est une vue en coupe selon B-B de la partie femelle du champ de bordure chirurgical représentée sur la figure 5,
- la figure 7 est une vue schématique montrant, en coupe, le dispositif de protection de l'invention mis en place contre les berges d'incision et équipé des éléments d'aspiration des secrétions d'incision.

### Modes de réalisation de l'invention.

Conformément à l'invention, l'agent antibactérien se caractérise par le fait qu'il comporte comme produit actif un tissu hémostatique de contact ayant un pH acide combiné à de la polyvidone iodée.

Cet agent antibactérien est destiné à être utilisé sur l'homme et/ou sur les animaux dans le but de protéger et/ou de traiter des plaies et/ou des incisions contre des infections bactériennes. L'utilisation de l'agent antibactérien est réalisée par voie locale en application sur la plaie et/ou les berges d'incision. On pourra par exemple utiliser un pansement chirurgical intégrant l'agent antibactérien conforme à l'invention.

La posologie sera adaptée en fonction du pH du tissu hémostatique de contact, de l'éventuelle infection bactérienne à traiter et du profil du patient. Pour avoir une efficacité maximale, l'agent antibactérien comporte avantageusement entre 70 µL et 130 µL de polyvidone iodée pure ou diluée, pour 2 cm² à 16 cm² de tissu hémostatique de contact. Toutefois, ces dosages ne sont pas limitatifs et pourront être amenés à varier selon les besoins de l'homme du métier et selon la concentration de polyvidone iodée (0,1 %, 1 %, 10 %, ...).

On utilise préférentiellement comme tissu hémostatique de contact de la cellulose oxydée régénérée ou non régénérée provenant de pâte de bois, du coton, de linters, de coton, de ramie, de jute, de papier ou de matériaux similaires, et aussi de cellulose régénérée. On pourra par exemple employer des produits connus sous le nom de : Surgicel®, Curacel®, Gelitacel®, OKCEL®, Resorcel®, Promogran®, etc.

La cellulose oxydée régénérée ou non régénérée est un hémostatique de contact résorbable sur le site d'application et dont l'action principale est mécanique. La cellulose oxydée régénérée est un polymère et plus particulièrement un polysaccharide formé de molécules de D-glucose. L'oxydation par le tétroxyde d'azote conduit à l'oxydation d'une partie des fonctions alcool primaire du glucose avec formation d'acide glucuronique. Le produit obtenu est un polymère de D-glucose et d'acide glucuronique.

À titre d'exemple, l'oxydation de la cellulose oxydée régénérée pour la fabrication du Surgicel® se fait de la manière suivante : l'oxydation est obtenue par réaction du tétroxyde d'azote en solution dans le perfluorohexane, les bobines sont ensuite lavées à l'alcool isoropylique à 50 % jusqu'à obtention d'un effluent de rinçage de PH> 3.1 Les bobines sont alors rincées deux fois par l'alcool isopropylique à 99 % puis séchées et emballées. Le séchage permet l'élimination de l'isopropanol. Les gazes sont entreposées dans une armoire ventilée en atmosphère contrôlée obtenue par une succession de montées en température accompagnées de mises sous vide partiel. La teneur en isopropanol résiduel est < 5 ppm. La phase de déshumidification aboutit à la quasi totale élimination de l'isopropanol. La cellulose oxydée non régénérée peut provenir de l'oxydation de fibres végétales et en particulier du coton (Gelitacel®, Okcel®,).

Dans une variante de réalisation, le tissu hémostatique de contact est de la cellulose oxydée régénérée ou non régénérée imprégnée de chitosane. Ce dernier est un polymère dérivé de la chitine, présent dans les crustacés et certains végétaux et champignons. On pourra par exemple employer des produits connus sous le nom de : Chitoflex®, Chitoskin®, BST-Demon®, etc.

Pour la polyvidone iodée, on pourra par exemple employer des produits connus sous le nom de : Bétadine®, Betaseptic®, Polydine®, Isodine®, Pevidine®, E-Z Scrub®, etc.

Afin de mettre en valeur l'effet synergique obtenu par la combinaison du tissu hémostatique de contact ayant un pH acide avec la polyvidone iodée, des tests ont été réalisés. Ces tests permettent d'évaluer sur différentes souches bactériennes :
- le pouvoir antibactérien d'un tissu hémostatique de contact ayant un pH acide seul,
- le pouvoir antibactérien de la polyvidone iodée seule,
- le pouvoir antibactérien d'un tissu hémostatique de contact ayant un pH acide combiné à de la polyvidone iodée.

### Méthodologie :

Selon la méthodologie de la norme NF EN ISO 20645 (août 2005).

### Matériels utilisés :

- Tissu hémostatique de contact ayant un pH acide :
o tissu de cellulose oxydée régénéré (COR) fabriqué par la société ETHICON® (SURGICEL®). 2 cm x 2cm en double épaisseur,
o tissu de cellulose oxydée non régénéré (CONR) fabriqué par la société GELITA MEDICAL® (GELITACEL®). 2 cm x 2cm en double épaisseur,

- Polyvidone iodée : Bétadine® Dermique à 10%. Dépôt de 93 µL sur les échantillons de tissu.
- Souches bactériennes :

| | | |
|---|---|---|
| o | Pseudomonas aeruginosa | CIP 103 467 |
| o | Staphylococcus aureus Méthicilline Résistant | CIP 103 811 |
| o | Staphylococcus aureus | CIP 4.83 |
| o | Escherichia coli | CIP 54 127 |
| ○ | Enterococcus hirae | CIP 58.55 |
| ○ | Enterococcus faecium vancomycine résistant génotype van A | CIP 106 915 |
| ○ | Enterococcus faecium vancomycine résistant génotype van B | CIP 104 676 |

- Milieu de gélose de dénombrement : tryptone caséine soja agar.
- Bouillon pour la préparation des inoculums : caséine soja.

### Protocole :

- Dépôt de 10 mL de gélose en surfusion dans une boite de Pétri et solidification à température ambiante.
- Dépôt du tissu à tester sur gélose :
   ○ Tissu soumis à l'essai.
   ○ Tissu témoin normalisé (neutre).
   ○ Tissu soumis à l'essai + Bétadine® Dermique à 10%.
   ○ Tissu témoin normalisé + Bétadine® Dermique à 10%.
- Préparation des inoculums contenant de 1 à 5 x 10⁸ UFC/mL pour chaque souche testée.
- Ensemencement de 1 mL d'inoculum dans 150 mL de gélose à 45°C.
- Dépôt de 5 mL de gélose ensemencée sur le tissu testé, laisser solidifier.
- Réalisation des essais en triplicata (tissu soumis à l'essai et tissu soumis à l'essai + Bétadine® Dermique à 10%) et un essai simple pour les témoins (tissu témoin normalisé et tissu témoin normalisé + Bétadine® Dermique à 10%).
- Incubation des géloses 24h à 37°C.
- Vérification de la croissance bactérienne et calcul de la largeur de la zone d'inhibition, c'est-à-dire la zone exemptée de bactéries.

L'évaluation de l'effet bactéricide repose sur l'absence ou la présence de croissance bactérienne dans la zone de contact entre la gélose et le tissu testé, et sur l'apparition éventuelle d'une zone d'inhibition autour du tissu testé.

### Résultats Tissu COR :

| Souches testées | Tissu testé | Zone d'inhibition (mm) | Croissance bactérienne | Évaluation de l'effet bactéricide |
|---|---|---|---|---|
| Pseudomonas aeruginosa CIP 103 467 | Tissu témoin | 0 | Importante | Insuffisant |
| | Tissu témoin + Bétadine® Dermique à 10%. | 0 | Importante | Insuffisant |
| | Tissu COR | 3 | aucune | Satisfaisant |
| | Tissu COR + Bétadine® Dermique à 10%. | 4 | aucune | Satisfaisant |
| Staphylococcus aureus CIP 4.83 | Tissu témoin | 0 | Importante | Insuffisant |
| | Tissu témoin + Bétadine® Dermique à 10%. | 0 | Importante | Insuffisant |
| | Tissu COR | 2,5 | aucune | Satisfaisant |
| | Tissu COR + Bétadine® Dermique à 10% | 4,2 | aucune | Satisfaisant |
| Escherichia coli CIP 54 127 | Tissu témoin | 0 | Importante | Insuffisant |
| | Tissu témoin + Bétadine® Dermique à 10%. | 0 | Importante | Insuffisant |
| | Tissu COR | 0,6 | aucune | Satisfaisant |
| | Tissu COR + Bétadine® Dermique à 10%. | 2,3 | aucune | Satisfaisant |
| Enterococcus hirae CIP 58.55 | Tissu témoin | 0 | Importante | Insuffisant |
| | Tissu témoin + Bétadine® Dermique à 10%. | 0 | Importante | Insuffisant |
| | Tissu de COR | 2 | aucune | Satisfaisant |
| | Tissu COR + Bétadine® Dermique à 10%. | 3,3 | aucune | Satisfaisant |
| Staphylococcus aureus Méthicilline Résistant CIP 103 811 | Tissu témoin | 0 | Importante | Insuffisant |
| | Tissu témoin + Bétadine® Dermique à 10%. | 0 | Importante | Insuffisant |
| | Tissu COR | 2,2 | aucune | Satisfaisant |
| | Tissu COR + Bétadine® Dermique à 10%. | 3,6 | aucune | Satisfaisant |
| Enterococcus faecium CIP 104 476 | Tissu témoin | 0 | Importante | Insuffisant |
| | Tissu témoin + Bétadine® Dermique à 10%. | 0 | Importante | Insuffisant |
| | Tissu de COR | 1,2 | aucune | Satisfaisant |
| | Tissu COR + Bétadine® Dermique à 10%. | 3,5 | aucune | Satisfaisant |
| Enterococcus faecium CIP 106 915 | Tissu témoin | 0 | Importante | Insuffisant |
| | Tissu témoin + Bétadine@ Dermique à 10%. | 0 | Importante | Insuffisant |
| | Tissu COR | 1,7 | aucune | Satisfaisant |
| | Tissu COR + Bétadine® Dermique à 10%. | 5,5 | aucune | Satisfaisant |

### Résultats Tissu CONR :

| **Souches testées** | **Tissu testé** | **Zone d'inhibition (mm)** | **Croissance bactérienne** | **Évaluation de l'effet bactéricide** |
|---|---|---|---|---|
| Pseudomonas aeruginosa CIP 103 467 | Tissu témoin | 0 | Importante | Insuffisant |
| | Tissu témoin + Bétadine® Dermique à 10%. | 0 | Importante | Insuffisant |
| | Tissu CONR | 0 | aucune | Satisfaisant |
| | Tissu CONR + Bétadine® Dermique à 10%. | 0,7 | aucune | Satisfaisant |
| Staphylococcus aureus CIP 4.83 | Tissu témoin | 0 | Importante | Insuffisant |
| | Tissu témoin + Bétadine® Dermique à 10%. | 0 | Importante | Insuffisant |
| | Tissu CONR | 0,5 | aucune | Satisfaisant |
| | Tissu CONR + Bétadine® Dermique à 10%. | 2,3 | aucune | Satisfaisant |
| Eschenchia coli CIP 54 127 | Tissu témoin | 0 | Importante | Insuffisant |
| | Tissu témoin + Bétadine@ Dermique à 10%. | 0 | Importante | Insuffisant |
| | Tissu CONR | 0 | aucune | Satisfaisant |
| | Tissu CONR + Bétadine® Dermique à 10%. | 1,7 | aucune | Satisfaisant |
| Enterococcus hirae CIP 58.55 | Tissu témoin | 0 | Importante | Insuffisant |
| | Tissu témoin + Bétadine® Dermique à 10%. | 0 | Importante | Insuffisant |
| | Tissu de CONR | 1,5 | aucune | Satisfaisant |
| | Tissu CONR + Bétadine® Dermique à 10%. | 3 | aucune | Satisfaisant |
| Staphylococcus aureus Méthicilline Résistant CIP 103 811 | Tissu témoin | 0 | Importante | Insuffisant |
| | Tissu témoin + Bétadine® Dermique à 10%. | 0 | Importante | Insuffisant |
| | Tissu CONR | 1 | aucune | Satisfaisant |
| | Tissu CONR + Bétadine® Dermique à 10%. | 3,2 | aucune | Satisfaisant |
| Enterococcus faecium CIP 104 476 | Tissu témoin | 0 | Importante | Insuffisant |
| | Tissu témoin + Bétadine® Dermique à 10%. | 0 | Importante | Insuffisant |
| | Tissu de CONR | 0,8 | aucune | Satisfaisant |
| | Tissu CONR + Bétadine® Dermique à 10%. | 3 | aucune | Satisfaisant |
| Enterococcus faecium CIP 106 915 | Tissu témoin | 0 | Importante | Insuffisant |
| | Tissu témoin + Bétadine® Dermique à 10%. | 0 | Importante | Insuffisant |
| | Tissu CONR | 2,2 | aucune | Satisfaisant |
| | Tissu CONR + Bétadine® Dermique à 10%. | 4 | aucune | Satisfaisant |

### Conclusion :

L'association du tissu de cellulose oxydée régénrée ou non régénérée avec de la Bétadine® Dermique à 10%, procure une inhibition bactérienne supérieure à la simple utilisation du tissu de cellulose oxydé (régénrée ou non régénérée) isolé ou de la Bétadine® Dermique à 10% isolée. Ces deux constituants agissent donc en synergie.

On pense que c'est le pH acide du tissu hémostatique de contact qui potentialise l'effet bactéricide de la polyvidone iodée.

Un autre aspect de l'invention concerne un dispositif de protection actif des berges d'incision destiné à éviter le développement des infections, comportant un champ de bordure chirurgical dans lequel est aménagée une fenêtre d'incision totale ou partielle, les bordures de ladite fenêtre d'incision intégrant un agent antibactérien conforme à l'invention.

Ce dispositif de protection est destiné à être utilisé lors d'opérations chirurgicales dans le but de protéger les berges d'incision. Il est mis en place après l'opération d'incision. L'incision à protéger peut également être une incision pour trocard dans le cadre d'une chirurgie laparoscopique.

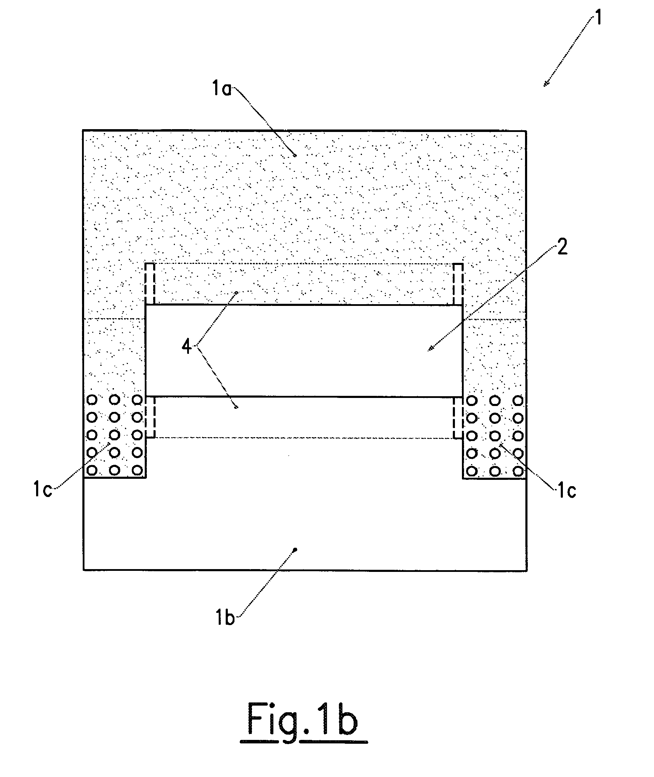

En se rapportant aux figures 1 a et 1 b, le dispositif de protection conforme à l'invention comporte un champ de bordure chirurgical 1 équipé d'une fenêtre d'incision 2, partielle ou totale, au travers de laquelle le chirurgien réalisera son intervention.

Le champ de bordure chirurgical 1 peut être réalisé en une seule pièce, mais est avantageusement formé d'une partie mâle 1a et d'une partie femelle 1 b séparables (figure 1a), la fenêtre d'incision 2 étant aménagée au niveau de la jonction entre ladite partie mâle 1a et ladite partie femelle 1b (figure 1b). L'utilisation d'un champ chirurgical en deux parties séparables simplifie sa conception et sa mise en place par le chirurgien.

Pour adapter la taille de la fenêtre d'incision 2 à la taille de l'incision à réaliser, la partie mâle 1a et la partie femelle 1b du champ de bordure chirurgical 1 sont maintenues en position par un dispositif de fixation réglable permettant d'ajuster la position de ladite partie mâle par rapport à ladite partie femelle et de faire varier la taille de ladite fenêtre d'incision.

En se référant aux figures 1a et 1b, le dispositif de fixation réglable est préférentiellement formé par des pattes de fixation 1 c adhésives agencées sur la partie mâle 1a et aptes à adhérer sur la partie femelle 1b. Les zones adhésives peuvent éventuellement être protégées par un papier de protection amovible que l'opérateur enlèvera juste avant la mise en place. Tout autre dispositif de fixation réglable équivalent peut être utilisé par l'homme du métier, par exemple des bandes à boucles et à crochets (VELCRO®), ou des éléments mâles s'insérant dans des éléments femelles.

Conformément aux figures 1 a, 1b et 2, la partie femelle 1b et la partie mâle 1a sont à bordures droites, avec des incisions 1d sur chaque coté aménagées de manière à ce que les bordures de la fenêtre d'incision 2 puissent être facilement introduites dans la berge chirurgicale tout en maintenant les pattes de fixation 1c à l'extérieur du corps du patient. Les incisions 1d sont avantageusement configurées de manière à ce qu'environ les 4/5^{ème} du champ puissent être introduits dans la berge chirurgicale. Selon une caractéristique avantageuse de l'invention, les incisions 1d sont prédécoupées de manière à ce que le praticien puisse introduire tout ou partie des bordures de la fenêtre d'incision 2 dans la berge chirurgicale, à sa convenance.

Le champ de bordure chirurgical 1 comporte une structure de base 3 réalisée dans un matériau stérile non tissé du type ouate de cellulose, mono-couche ou multi-couches. Tout autre matériau connu de l'homme du métier et convenant à la fabrication d'un champ de bordure chirurgical peut être employé. La structure de base 3 a une largeur comprise entre 20 cm et 40 cm, une longueur totale comprise entre 20 et 60 cm et une épaisseur variant de 1 mm à 2 mm. Toutefois, ces dimensions ne sont pas limitatives et peuvent être adaptées par l'homme du métier en fonction du type d'intervention chirurgicale réalisée.

Dans le but d'isoler efficacement le corps du patient de l'environnement extérieur et notamment de l'introduction d'un fluide contaminé, la face supérieure du champ de bordure chirurgical 1 est imperméabilisée. Cette imperméabilisation peut se faire au moyen d'un film plastique, d'un matériau imperméable aux liquides, d'un matériau hydrophobe ou tout autre matériau convenant à l'homme du métier.

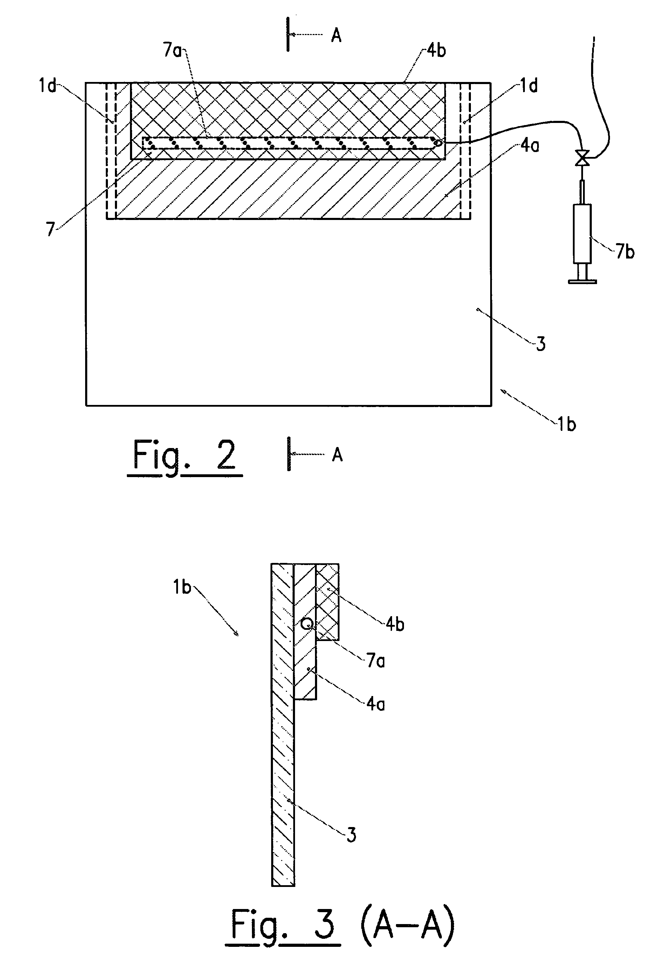

Sur les figures 2 et 3, seule est représentée la partie femelle 1b du champ de bordure chirurgicale 1. Toutefois, les caractéristiques représentées s'appliquent également à la partie mâle 1a ou au champ de bordure 1 dans le cas où celui-ci est réalisé en une seule pièce.

Conformément à l'invention et en se rapportant aux figures 1a et 1b, la face interne des bordures de la fenêtre d'incision 2 est recouverte d'un moyen 4 pour protéger les berges d'incision.

Conformément à l'invention, ce moyen de protection 4 comporte une couche 4b imprégnée d'un produit hémostatique antiseptique et/ou bactéricide et/ou antibactérien et/ou antibiotique et/ou présentant un pH acide.

Selon une caractéristique avantageuse de l'invention représentée sur la figure 2 et permettant d'optimiser la protection des berges d'incision, le moyen de protection 4 comporte une première couche 4a d'un matériau destiné à absorber les secrétions d'incision et la seconde couche 4b imprégnée d'un produit hémostatique antiseptique et/ou bactéricide et/ou antibactérien et/ou antibiotique et/ou présentant un pH acide.

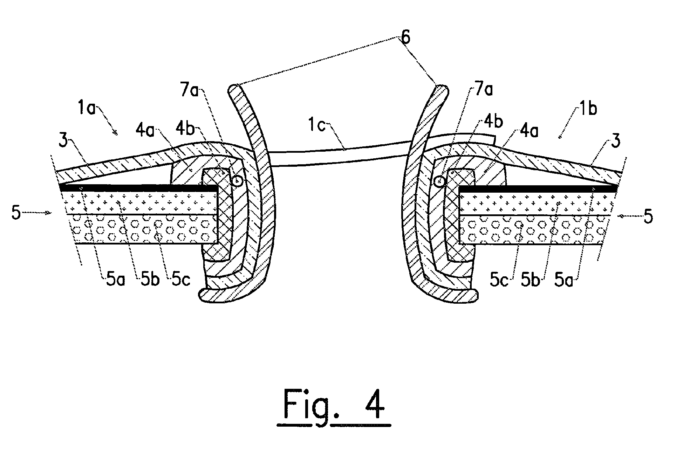

En se rapportant à la figure 4, la seconde couche 4b est aménagée de manière à ce qu'elle vienne au contact des berges d'incision 5 lors de la mise en position du champ de bordure chirurgical 1 autour de l'incision. Par « berges d'incision », on entend au sens de la présente invention le plan cutané 5a et/ou le plan sous-cutané 5b et/ou le plan osseux, musculaire ou aponévrotique 5c.

Selon une caractéristique avantageuse de l'invention représentée sur les figures, la première couche 4a et la seconde couche 4b sont disposées immédiatement adjacent aux bordures de la fenêtre d'incision 2 de façon à permettre au chirurgien de protéger rapidement les berges d'incision 5 par le simple rabattement desdites bordures dans le corps du patient.

Et dans le but de protéger parfaitement les berges d'incision 5, la seconde couche 4b est avantageusement aménagée de manière à ce qu'elle vienne en contact desdites berges et recouvre la partie supérieure et la partie inférieure de l'incision lors de la mise en place d'un écarteur 6. En effet, tout au long de l'intervention, ce dernier vient impacter et parfaitement maintenir en position le dispositif de protection objet de l'invention contre les berges 5, sans que les bordures de la fenêtre d'incision 2 viennent gêner le chirurgien.

La première couche 4a peut être de n'importe quel type conventionnel. Les matériaux absorbants couramment rencontrés sont par exemple la pâte de cellulose, des polymères hautement absorbants, des matériaux mousses absorbants, des fibres non tissées absorbantes. Il est possible de combiner ces différents matériaux de manière à avoir des propriétés différentes en ce qui concerne la capacité d'absorption de liquide, la capacité de répartition, et la capacité de stockage. Le matériau utilisé est de préférence, mais pas nécessairement, une compresse stérile épaisse absorbante de type HYDRE®.

La seconde couche 4b est préférentiellement réalisée par un tissu hémostatique de contact ayant un pH acide combiné à de la polyvidone iodée. On utilise préférentiellement comme tissu hémostatique de contact de la cellulose oxydée régénérée (Surgicel®, Curacel®, Resorcel®, Promogran®, etc) ou non régénérée (Gelitacel®, Curacel®, Okcel®). Dans une variante de réalisation, le tissu hémostatique de contact est de la cellulose oxydée régénérée ou non régénérée imprégnée de chitosane (Chitoflex®, Chitoskin®, BST-Demon®, etc). Pour la polyvidone iodée, on pourra par exemple employer des produits connus tels que Bétadine®, Betaseptic®, Polydine®, Isodine®, Pevidine®, E-Z Scrub®, etc.

Toutefois, la seconde couche 4b peut également être réalisée par l'intermédiaire d'une compresse stérile de type HYDREX® imprégnée d'un produit hémostatique antiseptique et/ou bactéricide et/ou antibactérien et/ou antibiotique et/ou présentant un pH acide. Tout autre matériau ou revêtement équivalent apte à être imprégné de tels produits peut être utilisé par l'homme du métier. Cette imprégnation a pour but d'éviter le développement des infections, des abcès de parois ou encore des hématomes. Parmi les produits d'imprégnation utilisables, on peut citer à titre d'exemple non limitatif : produits hémostatiques, peroxyde d'oxygène, antiseptiques à base d'ions d'Argent, alginate de Calcium, Piliostigma Reticulatum, les agents bactériostatiques, les agents bactéricides, les molécules antibiotiques, les molécules à pH acide, etc. Ces différents produits d'imprégnation peuvent être utilisés seuls ou en combinaison.

La seconde couche 4b vient couvrir totalement ou partiellement la première couche 4a. En se rapportant aux figures 2 et 3, la première couche 4a est plus large que la seconde couche 4b afin que ladite première couche puisse absorber et stocker efficacement les saignements ou autres sécrétions corporelles. En pratique, la première couche 4a a une longueur sensiblement égale à la largeur du champ de bordure chirurgicale 1 (et de la partie mâle 1 a et femelle 1 b), une largeur d'environ 10 cm et une épaisseur variant de 4 mm à 6 mm. La seconde couche 4b a également une longueur sensiblement égale à la largeur du champ de bordure chirurgicale 1 (et de la partie mâle 1a et femelle 1b), une largeur d'environ 5 cm et une épaisseur variant de 4 mm à 6 mm. Toutefois, ces dimensions ne sont pas limitatives et peuvent être adaptées par l'homme du métier en fonction le type d'intervention chirurgicale réalisée.

En se rapportant aux figures 2 à 4, la seconde couche 4b est avantageusement associée à un élément 7 permettant d'imprégner, à la demande, le tissu hémostatique de contact avec de la polyvidone iodée.

Cet élément 7 permet également d'imprégner la compresse stérile pouvant constituer la seconde couche 4b, avec un produit hémostatique antiseptique et/ou bactéricide et/ou antibactérien et/ou antibiotique et/ou présentant un pH acide.

En pratique, la seconde couche 4b est associée à une tubulure 7a reliée à un organe 7b permettant de distribuer, à la demande, de la polyvidone iodée (ou tout autre agent actif : hémostatique antiseptique et/ou bactéricide et/ou antibactérien et/ou antibiotique et/ou présentant un pH acide), ladite tubulure comportant dans sa partie distale des perforations configurées de manière à ce que la polyvidone iodée (ou tout autre agent actif) puisse imbiber ladite seconde couche.

La tubulure 6a est avantageusement placée entre la seconde couche 4b et la structure de base 3. Elle peut éventuellement être noyée dans la première couche 4a. Cette tubulure 7a permet une irrigation de la seconde couche 4b et donc du site d'incision opératoire. Cette tubulure 7a est perforée sur une partie distale, et sur sa partie proximale, elle se termine par une connectique de type Luer-lock® pouvant se connecter à une seringue ou tout autre organe de distribution 7b. En particulier, on peut prévoir d'utiliser à la place de la seringue, une pompe volumétrique configurée pour irriguer de manière continue et/ou à la demande du praticien, la tubulure 7a.

L'instillation d'un produit actif (polyvidone iodée ou autre), peut se faire par cette voie, venant ainsi imbiber la seconde couche 4b. Ces injections peuvent être répétées dans le temps, tant que le champ de bordure 1 est laissé en place.

La connectique permettant l'injection est préférentiellement située à droite de la partie mâle 1a et à gauche de la partie femelle 1b, de façon à ce que les deux voies d'injection se situent du même coté lorsque les deux parties sont mises en place sur chacune des berges chirurgicales d'incision 5 respectives. Une double voie en Y peut être connectée à ces deux sites d'injection afin de faciliter les instillations, et afin de pouvoir irriguer les deux parties 1 a et 1 b par un seul site d'injection.

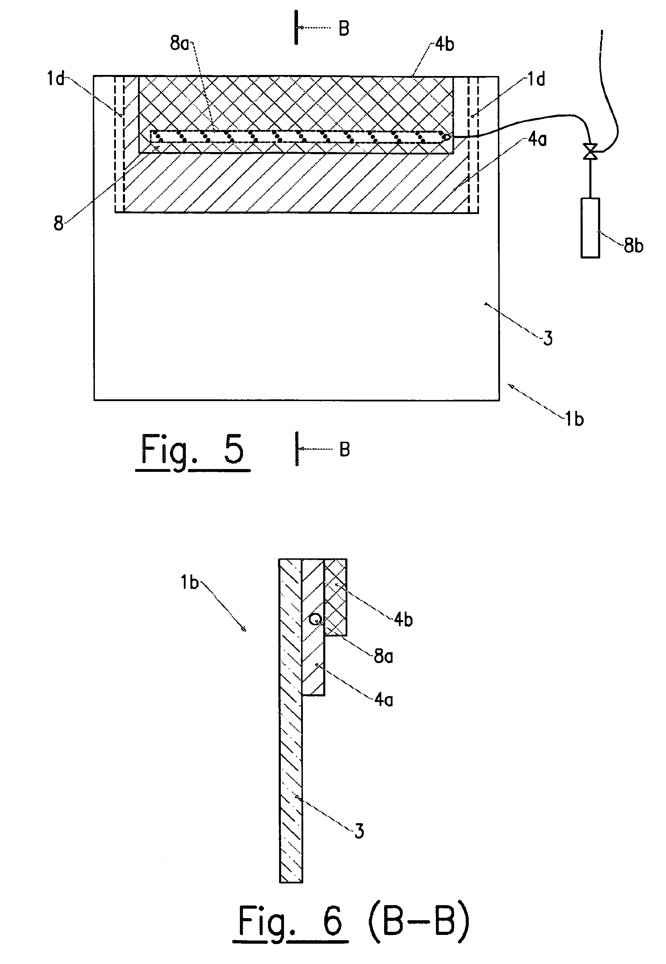

Sur les figures 5 et 6, seule est représentée la partie femelle 1b du champ de bordure chirurgicale 1. Toutefois, les caractéristiques représentées s'appliquent également à la partie mâle 1a ou au champ de bordure 1 dans le cas où celui-ci est réalisé en une seule pièce.

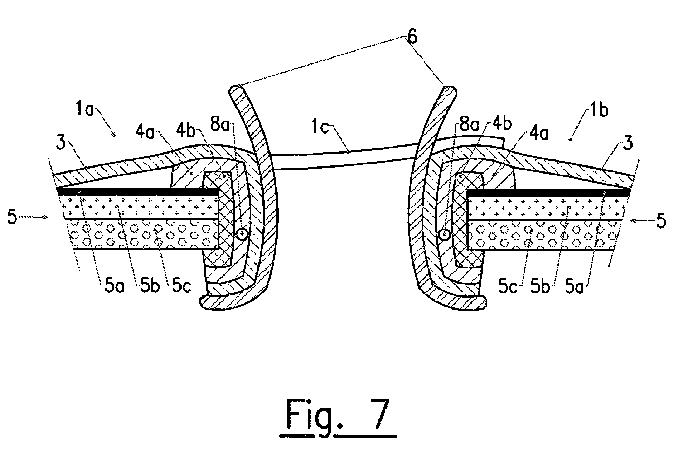

En se rapportant aux figures 5 à 7, la face interne des bordures de la fenêtre d'incision 2 peut être associée à un élément 8 permettant d'aspirer les sécrétions d'incision (saignements et/ou sécrétions corporelles et/ou magma adipocytaire libre et/ou d'éventuelles particules de talc de gant, ...) au niveau des berges d'incision 5. Le praticien a ainsi la possibilité d'assécher les berges d'incision 5 et d'évacuer de façon régulière les sécrétions du site opératoire.

Cette caractéristique doit être comprise comme étant indépendante de la conception du dispositif objet de l'invention (champ de bordure 1 réalisé en une seule pièce ou en deux pièces) et en particulier indépendante de la présence ou non de la seconde couche 4b et des propriétés antiseptiques ou non dudit dispositif.

En pratique, la première couche 4a destinée à absorber les sécrétions d'incisions est associée à une tubulure 8a reliée à un organe 8b permettant d'aspirer lesdites secrétions absorbées et donc d'assurer l'assèchement des berges d'incision 5.

Dans le cas où la première couche 4a est du type une compresse stérile épaisse absorbante, la tubulure 8a est avantageusement noyée dans ladite compresse. Si la première couche 4a est du type réalisée à partir d'un matériau mousse absorbant, la tubulure 8a pourra être noyée dans ledit matériau mousse ou être disposée contre une des faces de ladite première couche, l'aspiration créée par l'organe 8b permettant d'aspirer la totalité des saignements et/ou secrétions corporelles absorbés.

La tubulure 8a est avantageusement perforée sur une partie distale, et sur sa partie proximale, elle se termine par un embout destiné à se connecter à l'organe d'aspiration 8b. En pratique, on utilise une connectique de type Luer-lock® pouvant se connecter à une pompe ou tout autre organe d'aspiration 8b. En pratique, on peut utiliser une pompe électrique logée dans un boîtier portatif ou à une prise murale de vide. L'aspiration peut être réalisée de manière continue et/ou à la demande du praticien.

La tubulure 8a peut être simple ou multiple. Dans ce dernier cas, la tubulure 8a comporte plusieurs ramifications agencées de manière à homogénéiser l'aspiration des sécrétions d'incision absorbées par la première couche 4a.

La connectique permettant l'aspiration est préférentiellement située à droite de la partie mâle 1a et à gauche de la partie femelle 1b, de façon à ce que les deux voies d'aspiration se situent du même côté lorsque les deux parties sont mises en place sur chacune des berges d'incision 5 respectives. Une double voie en Y peut être connectée à ces deux sites d'aspiration afin de pouvoir aspirer les sécrétions d'incision au niveau des deux parties 1 a et 1 b par un seul site d'aspiration.

La tubulure 8a permettant d'aspirer les sécrétions d'incision absorbées par la première couche 4a peut être la même tubulure 7a permettant d'imprégner la seconde couche 4b d'un produit actif. Dans une variante de réalisation, les tubulures 7a et 8a sont distinctes.

## Revendications

1. Agent antibactérien comportant comme produit actif un tissu hémostatique de contact ayant un pH acide combiné à de la polyvidone iodée **se caractérisant par le fait que** le tissu hémostatique de contact est de la cellulose oxydée régénérée ou non régénérée.

2. Agent antibactérien selon la revendication 1, comportant entre 70 µL et 130 µL de polyvidone iodée pure ou diluée, pour 2 cm² à 16 cm² de tissu hémostatique de contact.

3. Agent antibactérien selon l'une des revendications précédentes, dans lequel le tissu hémostatique de contact est de la cellulose oxydée régénérée ou non régénérée imprégnée de chitosane.

4. Pansement chirurgical, **se caractérisant par le fait qu'**il intègre un agent antibactérien conforme aux revendications 1 à 3.

5. Dispositif de protection actif des berges d'incision (5) destiné à éviter le développement des infections, comportant un champ de bordure chirurgical (1) dans lequel est aménagée une fenêtre d'incision (2) totale ou partielle, **se caractérisant par le fait que** les bordures de ladite fenêtre d'incision intègre un agent antibactérien conforme aux revendications 1 à 3.

6. Dispositif selon la revendication 5, dans lequel :
- la face interne des bordures de la fenêtre d'incision (2) est recouverte d'une première couche (4a) d'un matériau destiné à absorber les secrétions d'incision et d'une seconde couche (4b) imprégnée d'un produit hémostatique antiseptique ayant pour but d'éviter le développement des infections,
- la seconde couche (4b) vient couvrir partiellement la première couche (4a), ladite première couche étant plus large que ladite seconde couche,
- la seconde couche (4b) est aménagée de manière à ce qu'elle puisse venir au contact des berges d'incision (5) et recouvre la partie supérieure et la partie inférieure de l'incision lors de la mise en place d'un écarteur (6),
- la seconde couche (4b) est un tissu de cellulose oxydée régénérée ou non régénérée combinée à de la polyvidone iodée.

7. Dispositif selon la revendication 6, dans lequel la seconde couche (4b) est associée à un élément (7) permettant d'imprégner, à la demande, le tissu hémostatique de contact avec de la polyvidone iodée.

8. Dispositif selon la revendication 7, dans lequel la seconde couche (4b) est associé à une tubulure (7a) reliée à un organe (7b) permettant de distribuer, à la demande, de la polyvidone iodée, ladite tubulure comportant dans sa partie distale des perforations configurées de manière à ce que la polyvidone iodée puisse imbiber ladite seconde couche.

9. Dispositif selon l'une des revendications 5 à 8, dans lequel les bordures de la fenêtre d'incision (2) sont aménagées de manière à ce qu'elles puissent venir au contact des berges d'incision (5), la face interne des bordures de ladite fenêtre étant recouverte d'une couche (4a) d'un matériau destiné à absorber les secrétions d'incision, ladite couche étant associée à une tubulure (8a) reliée à un organe (8b) permettant d'aspirer lesdites secrétions absorbées.

10. Dispositif selon la revendication 9, dans lequel la couche (4a) destinée à absorber les sécrétions d'incision est une compresse stérile épaisse absorbante, la tubulure (8a) étant noyée dans ladite compresse.

11. Dispositif selon la revendication 9, dans lequel la couche (4a) destinée à absorber les sécrétions d'incision est réalisée à partir d'un matériau mousse absorbant, la tubulure (8a) étant noyée dans ledit matériau mousse.

12. Dispositif selon la revendication 9, dans lequel la couche (4a) destinée à absorber les sécrétions d'incision est réalisée à partir d'un matériau mousse absorbant, la tubulure (8a) étant disposée contre une des faces de ladite couche (4a).

13. Dispositif selon l'une des revendications 9 à 12, dans lequel la tubulure (8a) est perforée sur une partie distale, et sur sa partie proximale, elle se termine par un embout destiné à se connecter à l'organe d'aspiration (8b).

14. Dispositif selon l'une des revendications 9 à 13, dans lequel la tubulure (8a) comporte plusieurs ramifications agencées de manière à homogénéiser l'aspiration des secrétions d'incision absorbées par la couche (4a).

## Claims

1. Antibacterial agent comprising, as active ingredient, a haemostatic contact cloth having an acidic pH combined with povidone iodine, **characterized in that** the haemostatic contact cloth is oxidized regenerated or non-regenerated cellulose.

2. Antibacterial agent according to Claim 1, comprising between 70 µL and 130 µL of pure or diluted povidone iodine, per 2 cm² to 16 cm² of haemostatic contact cloth.

3. Antibacterial agent according to either of the preceding claims, in which the haemostatic contact cloth is oxidized regenerated or non-regenerated cellulose impregnated with chitosan.

4. Surgical dressing, **characterized in that** it incorporates an antibacterial agent according to Claims 1 to 3.

5. Device for active protection of incision margins (5) intended for preventing the development of infections, comprising a surgical drape (1) in which a total or partial incision window (2) is arranged, **characterized in that** the borders of said incision window incorporate an antibacterial agent according to Claims 1 to 3.

6. Device according to Claim 5, in which:
- the internal face of the borders of the incision window (2) is covered with a first layer (4a) of a material intended to absorb the incision secretions and a second layer (4b) impregnated with an antiseptic haemostatic ingredient, the objective of which is to prevent the development of infections,
- the second layer (4b) partially covers the first layer (4a), said first layer being wider than said second layer,
- the second layer (4b) is arranged in such a way that it can come into contact with the incision margins (5) and covers the top part and the bottom part of the incision during the insertion of a retractor (6),
- the second layer (4b) is an oxidized regenerated or non-regenerated cellulose cloth combined with povidone iodine.

7. Device according to Claim 6, in which the second layer (4b) is coupled to an element (7) enabling impregnation, on demand, of the haemostatic contact cloth with povidone iodine.

8. Device according to Claim 7, in which the second layer (4b) is coupled to a tubing (7a) connected to a member (7b) enabling distribution, on demand, of the povidone iodine, said tubing comprising, in its distal part, perforations configured such that the povidone iodine can impregnate said second layer.

9. Device according to one of Claims 5 to 8, in which the borders of the incision window (2) are arranged such that they can come into contact with the incision margins (5), the internal face of the borders of said window being covered with a layer (4a) of a material intended to absorb secretions from the incision, said layer being coupled to a tubing (8a) connected to a member (8b) enabling aeration of said absorbed secretions.

10. Device according to Claim 9, in which the layer (4a) intended to absorb the secretions from the incision is a thick, sterile, absorbent compress, the tubing (8a) being embedded in said compress.

11. Device according to Claim 9, in which the layer (4a) intended to absorb the incision secretions is made of an absorbent foam material, the tubing (8a) being embedded in said foam material.

12. Device according to Claim 9, in which the layer (4a) intended to absorb the incision secretions is made of an absorbent foam material, the tubing (8a) being placed against one of the faces of said layer (4a).

13. Device according to one of Claims 9 to 12, in which the tubing (8a) is perforated on a distal part, and on its proximal part, it ends with a nozzle intended to be connected to the aspiration member (8b).

14. Device according to one of Claims 9 to 13, in which the tubing (8a) comprises several branches arranged so as to homogenize the aspiration of the incision secretions absorbed by the layer (4a).

## Patentansprüche

1. Antibakterielles Mittel, umfassend als Wirkstoff ein blutstillendes Kontaktgewebe mit einem sauren pH-Wert, kombiniert mit Polyvidon-Iod, **gekennzeichnet durch** die Tatsache, dass das blutstillende Kontaktgewebe regenerierte oder nicht regenerierte oxidierte Cellulose ist.

2. Antibakterielles Mittel nach Anspruch 1, umfassend zwischen 70 µL und 130 µL reines oder verdünntes Polyvidon-Iod pro 2 cm² bis 16 cm² blutstillendes Kontaktgewebe.

3. Antibakterielles Mittel nach einem der vorhergehenden Ansprüche, wobei das blutstillende Kontaktgewebe mit Chitosan imprägnierte, regenerierte oder nicht regenerierte oxidierte Cellulose ist.

4. Chirurgisches Verbandmittel, **gekennzeichnet durch** die Tatsache, dass es ein antibakterielles Mittel gemäß den Ansprüchen 1 bis 3 enthält.

5. Vorrichtung zum aktiven Schutz von Schnitträndern (5), die dazu bestimmt ist, das Entstehen von Infektionen zu verhindern, umfassend ein chirurgisches Abdecktuch (1), in dem ein Voll- oder Teilschnittfenster (2) angeordnet ist, **gekennzeichnet durch** die Tatsache, dass die Einfassungen des Schnittfensters ein antibakterielles Mittel gemäß den Ansprüchen 1 bis 3 enthalten.

6. Vorrichtung nach Anspruch 5, wobei:
- die Innenseite der Einfassungen des Schnittfensters (2) mit einer ersten Schicht (4a) aus einem Material, das dazu bestimmt ist, die Schnittsabsonderungen zu absorbieren und mit einer zweiten Schicht (4b), die mit einem antiseptischen, blutstillenden Produkt imprägniert ist, bedeckt ist, um das Entstehen von Infektionen zu verhindern,
- die zweite Schicht (4b) die erste Schicht (4a) teilweise bedeckt, wobei die erste Schicht größer ist als die zweite Schicht,
- die zweite Schicht (4b) derart angeordnet ist, dass sie mit den Schnitträndern (5) in Kontakt kommen kann und den oberen Teil und den unteren Teil des Schnitts bedeckt, wenn ein Wundhaken (6) angebracht wird,
- die zweite Schicht (4b) ein Gewebe aus regenerierter oder nicht regenerierter oxidierter Cellulose, kombiniert mit Polyvidon-Iod, ist.

7. Vorrichtung nach Anspruch 6, wobei die zweite Schicht (4b) mit einem Element (7) assoziiert ist, das es ermöglicht, das blutstillende Kontaktgewebe nach Bedarf mit Polyvidon-Iod zu imprägnieren.

8. Vorrichtung nach Anspruch 7, wobei die zweite Schicht (4b) mit einem Schlauchsystem (7a) assoziiert ist, das mit einem Organ (7b) verbunden ist, das es ermöglicht, nach Bedarf Polyvidon-Iod zu verteilen, wobei das Schlauchsystem in seinem distalen Teil Perforationen umfasst, die derart beschaffen sind, dass das Polyvidon-Iod die zweite Schicht durchtränken kann.

9. Vorrichtung nach einem der Ansprüche 5 bis 8, wobei die Einfassungen des Schnittfensters (2) derart angeordnet sind, dass sie mit den Schnitträndern (5) in Kontakt kommen können, wobei die Innenseite der Ränder des Fensters mit einer Schicht (4a) aus einem Material bedeckt ist, das dazu bestimmt ist, die Schnittabsonderungen zu absorbieren, wobei die Schicht mit einem Schlauchsystem (8a) assoziiert ist, das mit einem Organ (8b) verbunden ist, das es ermöglicht, die absorbierten Absonderungen abzusaugen.

10. Vorrichtung nach Anspruch 9, wobei die Schicht (4a), die dazu bestimmt ist, die Schnittabsonderungen zu absorbieren, eine absorbierende, dicke, sterile Kompresse ist, wobei das Schlauchsystem (8a) in die Kompresse eingebettet ist.

11. Vorrichtung nach Anspruch 9, wobei die Schicht (4a), die dazu bestimmt ist, die Schnittabsonderungen zu absorbieren, aus einem absorbierenden Schaummaterial ausgeführt ist, wobei das Schlauchsystem (8a) in das Schaummaterial eingebettet ist.

12. Vorrichtung nach Anspruch 9, wobei die Schicht (4a), die dazu bestimmt ist, die Schnittabsonderungen zu absorbieren, aus einem absorbierenden Schaummaterial ausgeführt ist, wobei das Schlauchsystem (8a) gegen eine der Seiten der Schicht (4a) ausgeformt ist.

13. Vorrichtung nach einem der Ansprüche 9 bis 12, wobei das Schlauchsystem (8a) an einem distalen Teil perforiert ist und an seinem proximalen Teil mit einem Aufsatz abschließt, der dazu bestimmt ist, mit dem Absaugorgan (8b) verbunden zu werden.

14. Vorrichtung nach einem der Ansprüche 9 bis 13, wobei das Schlauchsystem (8a) mehrere Verzweigungen umfasst, die derart gestaltet sind, dass sie das Absaugen der Schnittabsonderungen, die von der Schicht (4a) absorbiert werden, homogenisieren.
